# EUROPEAN PATENT SPECIFICATION

(11) **EP 1 800 699 B1**
(45) Date of publication and mention of the grant of the patent: **12.04.2017**
(21) Application number: 05785682.5
(22) Date of filing: 26.09.2005
(51) Int. Cl.: A61L 27/00, A61L 27/04, A61L 27/56, A61L 27/30, A61F 2/30, A61B 17/80, A61C 8/00

(54) **SCAFFOLD MATERIAL CAPABLE OF INDUCING BIOLOGICAL HARD TISSUE OR SOFT TISSUE**
GERÜSTMATERIAL ZUR INDUKTION VON BIOLOGISCHEM HARTEM GEWEBE ODER WEICHGEWEBE
MATERIAU D'ECHAFAUDAGE CAPABLE D'INDUIRE DES TISSUS BIOLOGIQUES DUR OU MOU

(30) Priority: 24.09.2004 JP 2004278152; 27.09.2004 JP 2004279979
(43) Date of publication of application: 27.06.2007
(73) Proprietor: HI-LEX Corporation, Takarazuka-shi, Hyogo 665-0845 (JP); Kuboki, Yoshinori, Sapporo-shi, Hokkaido 063-0038 (JP)
(72) Inventor: KUBOKI, Yoshinori, Sapporo-shi Hokkaido 063-0038 (JP); SEKI, Yasuo, Takarazuka-shi, Hyogo 665-0845 (JP); SHIOTA, Hiroyuki, Takarazuka-shi, Hyogo 665-0845 (JP)
(74) Representative: Hancox, Jonathan Christopher
(86) International application number: PCT/JP2005/017655
(87) International publication number: WO 2006/033435

(56) References cited:
- EP-A2- 0 178 650
- EP-A2- 1 245 670
- WO-A1-2004/012781
- WO-A2-01/68004
- WO-A2-03/007779
- WO-A2-2004/031361
- WO-A2-2005/114120
- FR-A1- 2 664 501
- JP-A- 2004 016 398
- JP-B- 6 016 800
- US-A- 4 439 152
- US-A- 5 397 359
- US-A- 5 397 359
- US-A- 5 416 022
- US-A- 5 961 328
- US-A1- 2003 232 124
- US-B1- 6 171 106
- B.M. KIM, S. SUZUKI, Y. NISHIRUMA,: "CELLULAR ARTIFICIAL SKIN SUBSTITUTE PRODUCED BY SHORT PERIOD SIMULTANEOUS CULTURE OF FIBROBLASTS AND KERATINOCYTES", BRITISH JOURNAL OF PLASTIC SURGERY, vol. 52, 1999, pages 573-578, XP002672316,

## Description

### Field of the Invention

This invention relates to a scaffold material capable of inducing a biological hard tissue or soft tissue used for an artificial tooth implant, an artificial joint implant, a bone fixation fixture such as a bone plate, and substitutional bone or an artificial organ and its holding apparatus. Further, this disclosure relates to a method for the culture of cells/microorganisms, to a method for producing biogenous substances (particularly, collagen for medical treatments), and to apparatuses used for these methods.

### Background Arts

[Patent Document 1] Japanese Published Patent Application No.H11-341
[Patent Document 2] Japanese Published Patent Application No.H8-140996
[Patent Document 3] Japanese Published Patent Application No.2004-67547
[Patent Document 4] Japanese Published Patent Application No.2002-67547

In the past, for an implant material to implant in a living body, implant materials capable of inducing a hard tissue such as an artificial tooth root, an artificial joint or an implant material capable of inducing a soft tissue such as an artificial internal organ and its holding apparatus, and a skin terminal were known. Here, for the bone fixation fixture such as the artificial tooth root, the artificial joint etc., the induction and growth of a bone tissue (osteoblast, osteocyte) which is the hard tissue in a living body is important. And for the artificial internal organ and its holding apparatus or the skin terminal, the induction and growth of the soft tissue (fibroblast) is important. As a metal material for the implant material, titanium and titanium alloy are preferably used because these materials have peculiar affinity with less foreign body reactions compared with other metals.

However, even if the foreign body reactions in a living body are few, when the implant material is implanted in a living body, fibroblasts etc. of the connective tissue system gather together with collagen fibers to form encapsulated tissues around the surface of the implant material. Therefore, because the implant material and the bone tissues cannot contact directly, and it takes long time to induce the bone tissues, it was not practical. Moreover, even the surface of the material is rough-finished plane, since it is two dimensional structures, it cannot connect with soft tissues including the encapsulated tissues etc. For that reason, in order to securely fix the implant material into a living body, it is necessary for the implant material and the biological hard tissue or soft tissue to have more mechanical connections in a short time period. For this purpose, implant materials having various geometrical structures provided with the portion mechanically connected with biological hard tissues or soft tissues have been developed.

In Patent Document 1, an implant material, which is covered by a thin layer of bubble structure made of metal whose outer surface has a plurality of aperture cells is disclosed. By impregnating the covered portion of the thin layer of bubble structure with reinforced bone cement, it presents an in-between mechanical property of the implant material and the bone.

In Patent Document 2, an implant material comprising an anchor portion formed by winding and compressing metal wires in multi-tiers in the lower periphery of the implant material is disclosed. This implant material also targets at increasing the metallically connected portion with the osteoblasts. And further, since the anchor portion excels at buffering function, it is hard to be broken against the external force in occlusion etc.

However in the implant material having the above described geometrical structure, the infiltration of osteoblasts was observed but the condition of osteointegration being integrated with cells was not satisfactory.

The inventor described that, " Osteoblasts preferably grow in a geometrical structure composed of thin fibers, and they show extremely high affinity to the geometrical structure composed of titanium fibers of the thickness less than 100 *µ* m having the expanse of 100 to 400 *µ* m, and have a nature to adhere positively thereto." in Patent Document 3. Based on this knowledge, a scaffold material capable of inducing a biological hard tissue comprising a rod made of titanium metal and a biological tissue implantation space formed by winding the layer which is formed by intertwining the titanium fiber or titanium base alloy fiber having diameter of less than 100 *µ* m, and having the aspect ratio of which is larger than 20 (minor axis: major axis ratio=1: larger than 20) is disclosed. The use of the scaffold material of Patent Document 3 will result in forming three dimensional physical connection between the material and osteoblasts resulting in extremely good osteointegration tissues.

However, the implant material equipped with the porous biological tissue implantation layer of Patent Document 3 is preferable in the mechanical connection with oseteoblasts, but it is required of further improvement in the durability against external forces, particularly the strength against applied forces to the joint surface between the rod and the hard tissue forming and inducing layer, and torsion, shear etc.

US 5,397,359 (Mittelmeier et al.) discloses a metal wire structure for endoprosthetics formed of a sintered hollow mesh knitting of elastic metal wires.

This invention is directed to improve the strength against applied forces to the joint surface between the rod and the hard tissue forming and inducing layer, and torsion, shear etc., while preserving the geometrical space in which hard tissues (osteoblasts) grow preferably.

At present, when an artificial internal organ commencing with an artificial heart is implanted in a living body, in order to hold the artificial internal organ, a separate artifact being easy to connect with biological tissues is implanted together with the artificial internal organ. This invention is also directed to provide a scaffold material and implant material whose pore size is easy to induce soft tissues and has geometrical spaces, and is used for skins and muscles which are various kinds (anchorage dependent) cells or soft tissues other than osteoblasts.

### Disclosure of invention

The scaffold material capable of inducing biological tissue of this invention (claim 1) comprises a metal substrate and a metal fiber layer composed of a metal wire prepared in the outer periphery of the metal substrate, wherein the average pore size of the metal fiber layer is 100 to 400 *µ* m. The scaffold material further comprises a binding layer composed of a metal wire prepared in an inner periphery of the metal fiber layer, which has the average pore size of less then 100 *µ* m. Further, the average pore size of the metal fiber layer is preferable made to vary at a slant or in incremental steps from the innermost side to the outermost side (claim 2).

As the metal fiber layer of the above scaffold material, a metal nonwoven fabric formed by intertwining metal wires of the diameter 5 to 400 *µ* m maybe used(claim 3). Further the metal fiber layer maybe composed of metal wires having the diameter of 5 to 400 *µ* m and the average pore size formed at a slant or in incremental steps maybe made according to the difference of the degree of intertwining or the difference of the diameter of the metal wires. (claim 4, 5)

Further, the binding layer maybe formed by intertwining metal wires of diameter 5 to 400 *µ* m, and the metal powders or metal particles maybe implanted between the intertwined wires, forming a binding layer having the pore size of smaller than 100 *µ* m (claim 6). The diameter of the metal powders or the metal particles is preferable to be 100 *µ* m or less (claim 7).

Further the reinforcing layer composed of a metal wire having a diameter of 100 to 2000 *µ* m maybe formed at the periphery of the metal fiber layer where the average pore is made to be 100 to 2000 *µ* m may be formed(claim 8).

The metal substrate of such scaffold material is made to be a rod in shape based on the usage, and a bridge girder or a protrusion may be formed on the surface of the rod protruding outside against the radial direction of the rod (claim 9). And, the metal substrate maybe formed in a plate shape and a protrusion protruding outward in the plate surface may be formed (claim 10).

Further, the scaffold material is preferable to be formed by sintering all the metal substrate and the metal fiber layer and/or the binding layer together (claim 11). Such sintering is preferable to be performed at a temperature 0.3 to 0.9 times of the melting point (Tm · °C) of the metal substrate (claim 12). However, the metal substrate and the metal fiber layer or the binding layer may be bonded together with an adhesive material (claim 13). The material of the metal substrate and the metal fiber layer and/or the binding layer is preferable to be the one or two kinds of metal selected from a group composed of titanium, titanium alloy, gold, gold alloy (claim 14), and the metal fiber layer and/or the binding layer is preferable to be treated by apatite formation liquid and coated by hydroxyapatite containing apatite carbonate or other calcium phosphate compounds (claim 15).

As described above, the metal fiber layer and or binding layer which is coated or not coated with calcium phosphate compound, is preferable to be coated by one or two kinds or more of materials selected from groups composed of cytokine such as bone morphogenetic protein etc., hormone such as insulin etc., micro active substance such as interferon etc (claim 16).

The biomedical implant material of this invention comprises a scaffold material capable of inducing a biological hard tissue or soft tissue implanted in a living body and integrally fixed to the peripheral surface of the living body (claim 17). The implant material is preferable to be used for bone fixation fixtures such as artificial tooth roots, artificial joints, and bone plates, substitutional bones, artificial internal organs or their holding apparatuses, and skin terminals (claim 18).

Since the scaffold material (claim 1) is provided with the metal fiber layer whose average pore size is 100-400 *µ* m, it is excellent in inducing biological hard tissue and implantation property. Further, osteoblasts are preferably comes to stay and are easy to grow up. Therefore, the osteoblasts and the scaffold material are integrated to form as it is called collaboration.

The scaffold material of above and provided with the binding layer whose average pore size is less than 100 *µ* m inside of the metal fiber layer, the adhesiveness of the metal substrate and the binding layer is excellent, and they are adhered firmly. So, the junction strength of the metal fiber layer and the metal substrate becomes stronger interposing the binding layer.

Further, since the average pore size of the metal fiber layer are made to vary at a slant or in incremental steps increasing from the innermost layer to the outermost layer, it is hard to be sheared when forces are applied to the scaffold material. This is because the interface of the both layers disappears after the growth of the osteoblasts. And further, cells are easy to infiltrate because the average pore size of the outermost portion is largest.

The metal fiber layer formed by fixing a metal nonwoven fabric formed by intertwining metal wires of the diameter 5 to 400 *µ* m (claim 3), and the metal fiber layer composed of metal wires having the diameter of 5 to 400 *µ* m whose average pore size is made to vary at the slant or in incremental steps according to the difference of the degree of intertwining of the metal wires or according to the difference of the diameter of the metal wires (claim 4, 5) are easy to manufacture.

For example, followings can be cited; the metal nonwoven fabric composed of the same diameter wires or the wires having same diameter are gradually winded and/or intertwined with gradually lessened force; a plurality of metal nonwoven fabrics of different average pore sizes is continually winded; metal wires or metal nonwoven fabrics are winded or encased in a metal mold and different pressures are applied from outside to provide difference in the degree of intertwining; furthermore, metal wires of different diameter or metal nonwoven fabrics composed of different diameter metal wires are intertwined and/or winded with a constant force. As a matter of course, these may be combined, but not limited to these.

In the case that the binding layer is formed by intertwining metal wires of diameter 5 to 400 *µ* m and metal powders or metal particles is thickly implanted between the intertwined wires (claim 6), since the average pore size of the metal fiber layer is freely adjustable before and after the provision of the metal wires in the metal substrate, it is easy to manufacture. Moreover, the average pore size of the metal fiber layer may be adjusted by filling in metal powders or metal particles, and the pore size of the metal fiber layer may be made uniformly, step-by-step, or incliningly.

In the scaffold material of this invention where the reinforcing layer composed of metal wires of the diameter 100 to 2000 *µ* m are prepared on the outer periphery of the metal fiber layer, and the reinforcing layer is formed have the average pore size of 100 to 2000 *µ* m (claim 8), this reinforcing layer becomes the frame of the entire scaffold material, further strengthening against external forces such as torsion.

By making the metal substrate be a rod in shape, it can be used as an artificial tooth, an artificial joint, a substitutional bone. By making the metal substrate be a plate in shape, it can be used as a bone fixation fixture such as a bone plate, an artificial organ or a holding apparatus. Further, in the case that a bridge girder or a protrusion extending outward against the radial direction of the rod is formed on the surface of the rod (claim 9, 10), the adhesive strength of the metal substrate and the metal fiber layer against external forces particularly against torsion and shearing can be enhanced.

In the case that the metal substrate and the metal fiber layer and/or the binding layer of the scaffold material are formed by sintering together (claim 11), the jointed portion of the metal substrate and the metal wires and the mutual intertwined portion of the metal wires are adhered by sintering, it can be further strengthened. In such a case, the metal substrate and the metal wires are preferable to be the same kind of metal.

In the case that the material of the metal substrate and the metal fiber layer and/or the binding layer of the scaffold material of this invention is titanium, titanium alloy, gold, gold alloy (claim 14), it is preferable because the biocompatibility is high. Moreover, in the case that the metal fiber layer and/or the binding layer is treated by apatite formation liquid and coated by hydroxyapatite containing apatite carbonate or other calcium phosphate compounds and cytokine such as bone morphogenetic protein, hormone such as insulin, micro active substance such as interferon (claim 15), the osteoblasts can be induced into the metal fiber layer within a short time period and can be implanted.

### Brief Description of Drawings

[Figure 1] Figure 1 is a cross section showing an embodiment of the scaffold material of this invention.
[Figure 2] Figure 2 is a perspective view showing the other embodiment of the scaffold material of this invention.
[Figure 3] Figure 3 is a perspective view showing further the other embodiment of the scaffold material of this invention.
[Figure 4] Figure 4 is a microphotograph showing the scaffold material of this invention after the culture of cells in the metal fiber layer.
[Figure 5] Figure 5a is a side cross section showing further the other embodiment of the scaffold material of this invention, and Figure 5b is a front cross section thereof, and Figure 5c is a side view showing the substrate.
[Figure 6] Figure 6 is a side cross section showing further the other embodiment of the scaffold material of this invention.
[Figure 7] Figure 7 is a side cross section showing further the other embodiment of the scaffold material of this invention.
[Figure 8] Figure 8 is a side cross section showing further the other embodiment of the scaffold material of this invention.
[Figure 9] Figure 9a is an outline drawing showing the medical treatment using the scaffold material of Figure 8.
[Figure 10] Figure 10 is a table showing the comparison of the breaking torque of the scaffold material of this invention and the conventional publicly known scaffold material.
[Figure 11] Figure 11 is a table showing the comparison of the breaking torsion angle of the scaffold material of this invention and the conventional publicly known scaffold material.
[Figure 12] Figure 12 is an outline configuration drawing of a production apparatus for a biogenous substance.
[Figure 13] Figure 13 is a perspective view showing the state of the holder and the culture platform before assembling in Figure 12.
[Figure 14] Figure 14 is a cross section of the holder and the culture platform of Figure 13 after assembling.
[Figure 15] Figure 15 is a substantial process drawing showing a production apparatus.
[Figure 16] Figure 16a and Figure 16b is a cross section showing a culture platform.
[Figure 17] Figure 17 is a table showing the production amount of collagen produced by the described culture method and the conventional culture method.

### Best mode for carrying out the invention

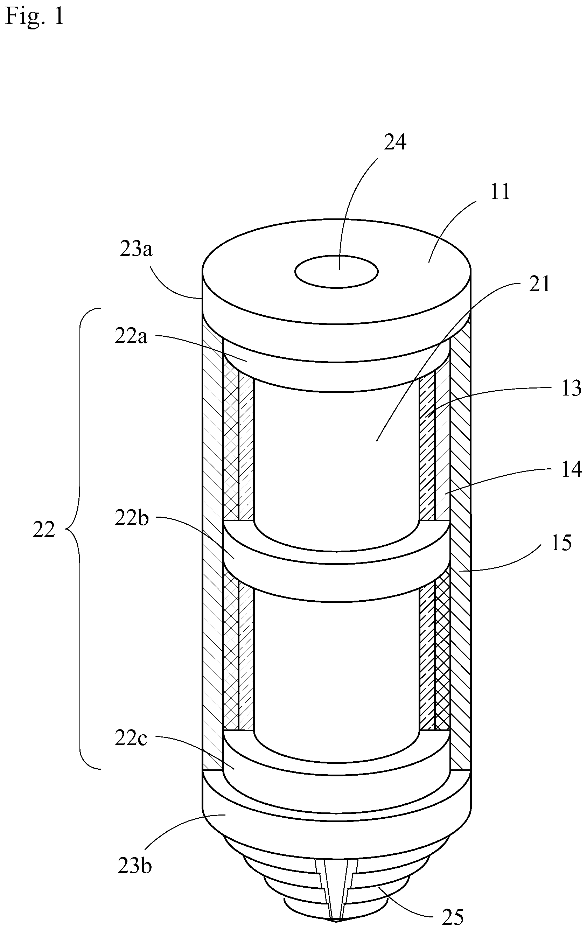

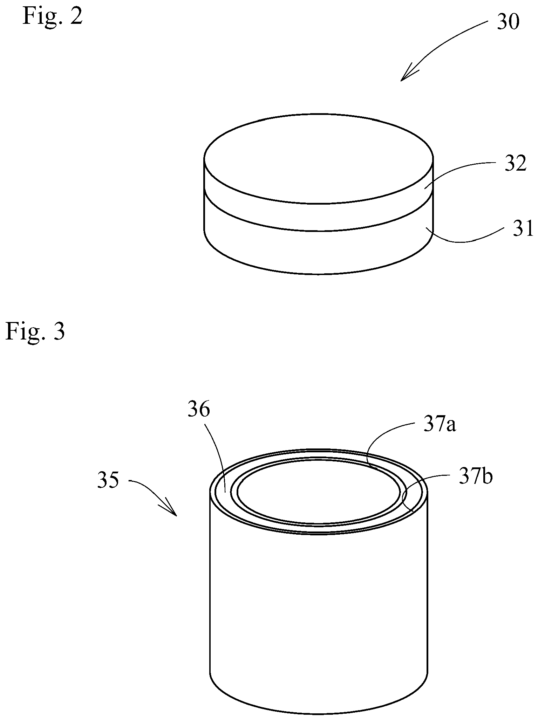

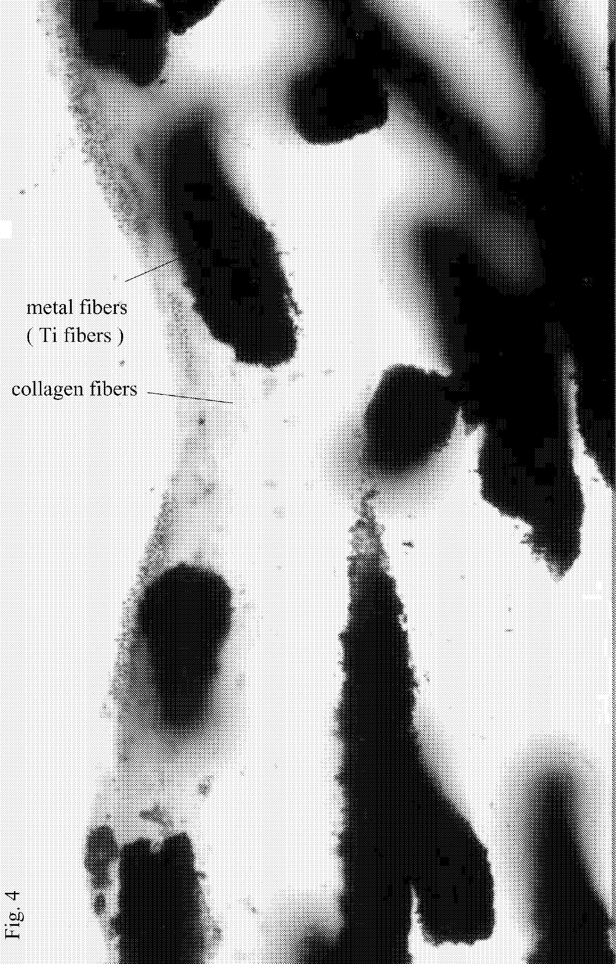

Figure 1 is a cross section showing an embodiment of the scaffold material of this invention, Figure 2 is a perspective view showing the other embodiment of the scaffold material of this invention, Figure 3 is a perspective view showing further the other embodiment of the scaffold material of this invention, Figure 4 is a microphotograph showing the scaffold material of this invention after the culture of cells in the metal fiber layer. These embodiments are disclosed giving an actual example as a help for facilitating this invention, but this invention is not limited to these.

The artificial tooth root 10 of this invention comprises, a rod 11 as the metal substrate, a binding layer 13 formed in the outer periphery of the rod, a metal fiber layer 14 provided in the outer periphery of the binding layer, and a reinforcing layer 15 provided in the outer periphery of the metal fiber layer for supporting the whole as a frame.

The rod 11 is composed of a body 21 whose diameter is 3-5mm, bridge girders 22a, 22b, 22c provided in the upper, middle, lower part of the body, and flange portions 23a, 23b provided in the upper and lower end of the body. In the upper face of the upper flange portion 23a, an insetting hole 24 for insetting an anchor portion (not shown in drawings) of tooth is formed. The lower part of the lower end flange portion 23b is made to be screw tap so as to facilitate insertion to bone tissues. The whole length of the rod 11 is 7-20 mm, and the height of the bridge girder 22 is preferable to be 0.2-2 mm. As such rod 11, what is made of titanium, titanium alloy, gold, gold alloy is preferable. However, it is not particularly limited so far as it has high biocompatibility such as stainless steel or cobalt-chrome alloy etc.

The binding layer 13 is formed by tightly winding a metal wire having the diameter of 5-400 *µ* m between the bridge girders 22 on the outer periphery of the rod 11, so as to make the thickness be 0.1-2 mm. The metal fiber layer 14 having an average pore size of 100 to 400 *µ* m is formed by wrapping a reticulated web (nonwoven metal fabric) of intertwined metal wires having diameter of 5-400 *µ* m around the outer periphery of the binding layer 13, so as to make the thickness be 0.3-10 mm. After that, the intersecting points of the each metal wire and the contact point of the binding layer and the rod are fusion bonded by vacuum sintering together with the rod. As the fixing method of this metal wire and metal nonwoven fabric, the molding method is employed. More specifically, the volume in which metal wires and metal nonwoven fabric are accommodated is calculated, and then from the void ratio and the wire diameter, the volume and weight of the metal wires and metal nonwoven fabric to be used are calculated. The required amount is pushed in between the substrate and the mold, and vacuum sintered under the pressure while the thickness being calculated. By making the whole thickness of the metal fiber layer and the binding layer be 0.4-12 mm, the vacuum sintering of the web composed of metal wires can be facilitated. The sintering temperature is different depending on the melting point of the metal wire (Tm·°C), and it is preferable to be 0.3-0.9 times of the melting point of the metal and metal alloy (Tm·°C). For example, when titanium is used as the substrate and metal wire, since the melting point of the titanium is 1668°C, the sintering temperature is preferable to be about 500-1500°C.

The reinforcing layer 15 is the layer in which a reticulated web of metal wires of the diameter 100-2000 *µ* m being intertwined to make the average pore size be 100-2000 *µ* m is wrapped around the metal fiber layer 14 so as to be the thickness 0.1-5 mm.

Since the reinforcing layer 15 is thus formed with comparatively thick metal wires, it functions as a frame for supporting the whole scaffold material, preventing the interface between the metal layers of outer most layer and intermediate layer, and the living body from breaking by external forces such as torsion or shearing.

On each layer, an inducing agent to induce living cells is coated. As such inducing agent, hydroxyapatite containing apatite carbonate, and other calcium phosphate compounds etc. can be cited. Thereby, the collaboration zone which is the integration of bone tissues produced by osteoblasts and metal fiber layer 14 can be attained within a shorter time period. Further, since the pore size of the reinforcing layer is larger than the average pore size of the metal fiber layer, growth of the osteoblasts up to the metal fiber layer is not disturbed.

The artificial tooth root 10 thus configured is implanted in the implant portion of the jaw bone formed by drilling. Thereby, the living body tissues reaches the metal fiber layer 14 and the binding layer 13 through the reinforcing layer 15 where the average pore size is relatively large. Since, the metal fiber layer 14 is equipped with a geometrical structure for which osteoblasts have a preference, osteoblasts grow within a short period. The period to come into the osseointegration condition which is the integrated condition of the metal fiber 14 and the osteoblasts is about 1 to 2 months though differences among individuals are observed.

Thus, the osteoblasts take roots in every layer to form bones, and furthermore, being metabolizing, the binding strength of the artificial tooth root 10 always contributes largely. Even when external forces by dental occlusion etc. (press, torsion, shearing) affect, the existence of the binding layer 13 prevents the interface between the metal fiber layer 14 and the substrate from breaking. In other word, once the osteoblasts grow, the breaking in the interface between the living body and the metal fiber layer 14 will not likely to occur.

The each layer of the artificial tooth root 10 of Figure 1 is formed by vacuum sintering the intertwined metal wires. However, metal powders or metal particles having the diameter of less than 100 *µ*m with the same quality of nonwoven fabrics may be fixed around the outer periphery of the body of the rod by using mold and then vacuum sintering. And, epoxy adhesive agent, UV cure adhesive etc. is coated, and a web composed of metal wires of the diameter 5-500 *µ*m may be wrapped around the outer periphery of the rod. Since the each layer is thus formed, the rod is firmly fixed to the metal fiber layer 14 through the binding layer 13. But in the case that all the layers are composed of the same metal or metal alloy of the rod and fixed to the rod by sintering together, it is most preferable because the rod and the metal fiber layer can be integrated by fusion bonding.

The scaffold material 30 of Figure 2 comprises a titanium plate 31 and a metal fiber layer 32 provided on the upper face. The metal fiber layer 32 is the layer in which a metal nonwoven fabric of the average pore size 100-400 *µ* m is firmly fixed to the titanium plate 31. This scaffold material is attached to an artificial internal organ and implanted in a living body. Thereby, in the metal fiber layer, soft tissues grow in about 1 to 2 months to hold the artificial internal organ firmly.

The scaffold material 35 of Figure 3 comprises a cylindrical titanium rod 36 and a metal fiber layer 37a, b of the average pore size 100-400 *µ* m provided inside and outside thereof. Such scaffold material 35 can be used by being implanted in a living body as artificial blood vessels and other regeneration tubes for esophagus, trachea, nerve, vertebrae.

The photograph shown in Figure 4 shows the binding of the metal fibers and the collagen fibers synthesized by the above cells after the cell MCT3T3-E1 is cultured in four weeks and in six weeks on the titanium nonwoven fabric of the average pore size 100-300 *µ*m. As shown in Figure 2, in the brief period of one month, the metal fiber and the collagen fiber are found to be combined closely and soft tissues to be induced and to grow.

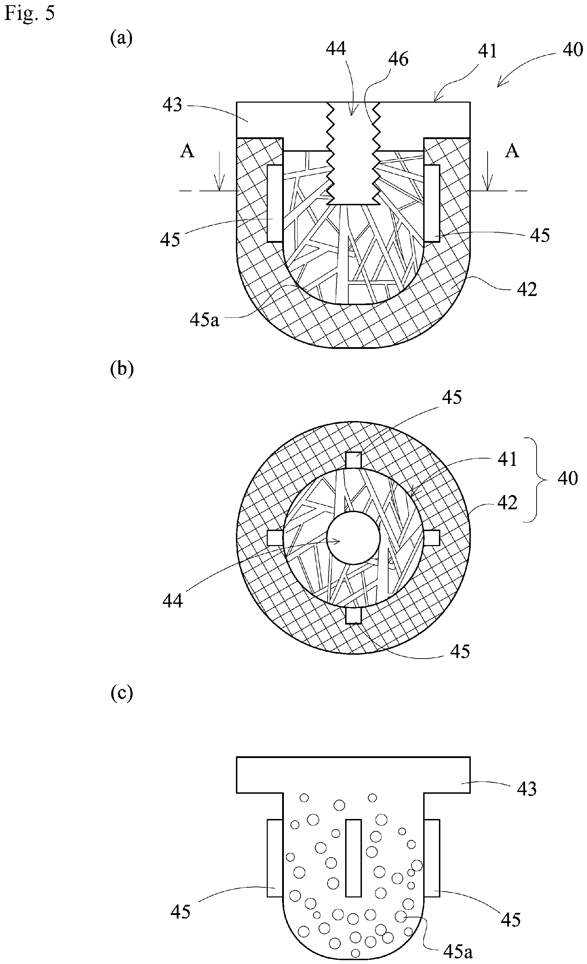

The scaffold material shown in Figure 5a is used for the artificial tooth root capable of inducing a biological hard tissue. This artificial tooth root 40 comprises a cylindrical substrate 41 and a cell induction layer 42 provided in the outer periphery of the substrate.

The substrate 41 have a flange portion 43, a lead-in hole 44 formed at the center of the upper face toward the center along the column axis, and a protrusion 45 protruding from the side face so as to extend toward the axial direction. The substrate 41 is a porous structure having microscopic holes 45a of the diameter 2mm or less, and the lead-in hole 44 and outside of the substrate are intricately communicated through the microscopic holes. Inside of the lead-in hole 44, a female screw 46 is formed to screw together with an upper structure or an abutment (not shown in the figure) to which the artificial tooth root is mounted.

In the case that metal is used as the material of such substrate, the following forming methods can be employed, for example; metal fibers are charged in a mold, solidified, and vacuum sintered to shape; melted metal is inpoured to a mold, and gas bubbles are generated accompanying the precipitation of supersaturated gas atom in solidification; or the outer shape is arranged in solidification and perforated by drill etc. and shaped. Further, in the case that synthetic resin is used as the material of the substrate, melted synthetic resin with foaming agent is inpoured to a mold and shaped.

The cell induction layer 42 is a porous structure having the average pore size of 100-400 *µ* m and provided so as to contact to the flange portion 43 and the protrusion 45. Thereby, it is constructed so that the cell induction layer does not easily depart from the substrate even external forces are applied. Moreover, such cell induction layer 42 is processed by apatite forming liquid, and coated by hydroxyapatite, other calcium phosphate compounds, cytokine as bone morphogenetic protein etc., hormone as insulin etc., micro active substance as interferon etc. Thereby the induction of biological hard tissues can be enhanced.

Such cell inducing layer 42 is formed, for example, by wrapping metal nonwoven fabrics formed by intertwining metal wires of diameter 5-400 *µ* m, so as to form the pore size of 100-400 *µ* m. And when synthetic resin is used, it may be formed by molding and may be fitted to the substrate 41 afterward.

Such artificial tooth root 40 is mounted in the mouth as follows. First of all, the bone marrow liquid etc. is injected into the lead-in hole 44 of the artificial tooth root 44 (mounting portion of the abutment), then, the lead-in hole 44 is pressed to be positive pressure by an injection syringe to feed the bone marrow liquid into inside of the substrate 41. Afterward, the artificial tooth root 40 is implanted into the mouth, a cover having male screw or a cover to close the lead-in hole 44 is mounted to the lead-in hole 44. The tooth 40 is left as it is for a certain period until the artificial tooth 40 is integrated with the living body. In this period, the bone marrow liquid reaches the cell induction layer 42 through the microscopic holes. Therefore, the regeneration of the bone begun from outside of the artificial tooth root is enhanced. After the certain period, the cover is removed and the lead-in hole is cleaned, and then an abutment having male screw is attached to the lead-in hole 44.

Thus, such treatment using the artificial tooth root 40 stays unchanged against conventional treatment operations. However, it is equipped with the cell induction layer 42 composed of the porous structure having the average pore size of 100-400 *µ* m, and the bone marrow liquid is injected into the lead-in hole 44 to distribute the bone marrow to whole of the substrate, the regeneration rate of the bone is especially improved by the bone marrow effect.

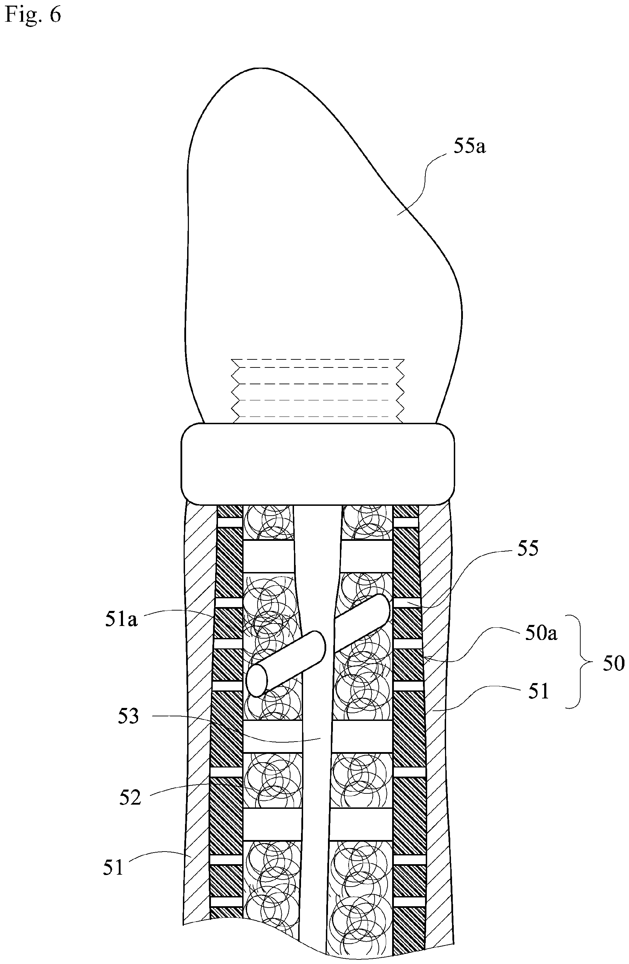

The scaffold material shown in Figure 6 is used in the artificial tooth root capable of inducing a biological hard tissue, and this artificial tooth root 50 comprises a cylindrical substrate 50a, and a cell induction layer 51 provided in the periphery of the substrate.

The substrate 50a comprises a substrate body 51a and a three dimensional porous structure 52 to be charged therein, and a supporting column 53 disposed therein.

In the side face of the cylindrical substrate body 51a, a small hole 55 of the diameter 2 mm or less is formed. This substrate body 51a is manufactured so that; the substrate body 51a is formed by side perforation of the cylindrical metal body. The supporting column 53 is inserted inside of the substrate body for sintering; after charging the metal fibers into the substrate body, the three dimensional porous structure 52 is formed by sintering operation.

The three dimensional porous structure 52 is not particularly limited, and the three dimensional porous structure supplies nutrition for bone marrow etc. to the cell induction layer 51 through substrate 51a as described later. And, since, blood vessels and nerves are also brought in and formed inside of the three dimensional porous structure 52, tooth pulp, as it is called, can be formed. Such three dimensional porous structure 52 is formed by charging synthetic fiber of synthetic resin or metal having biocompatibility into the substrate body.

And, the supporting column 53 is disposed so as to keep the strength of whole of the artificial tooth root. For such supporting column, a material having biocompatibility is used.

The cell induction layer 51 is substantially same with the cell induction layer 42 of Figure 42, and formed by wrapping, for example, nonwoven fabrics formed by intertwining metal wires of the diameter 5-400 *µ* m to make the pose size be 100-400 *µ* m.

Since the artificial tooth root 50 is thus configured, it can be mounted in the mouth by the same procedure with the artificial tooth root 40 as follows; first, the bone marrow liquid etc. is injected into inside of the three dimensional porous structure 52 which is the inside of the substrate of the artificial tooth root, after that, the inside of the substrate body is made to be positive pressure by an injection syringe, so as to distribute the bone marrow averagely. And then, the artificial tooth root 50 is implanted into the mouth, and a cover (not shown in the figure) is mounted to the opening of the substrate, and is left as it is for a constant period until the artificial tooth 50 is integrated with the living body. In this period, the bone marrow liquid charged inside of the substrate reaches, same as the artificial tooth root 40, the cell induction layer 51 through the perforation 55 of the substrate body. Therefore, the regeneration of the bone begun with the inside is enhanced. After that, the cover is removed and the lead-in hole is cleaned, and then an abutment 55a is attached.

Since the three dimensional porous structure 52 is thus equipped inside of the substrate, the shortest nutrition supply root (artificial tooth pulp) for supplying the nutrition stored in the three dimensional porous structure 52 is formed. By this artificial tooth pulp, the regeneration of dentine and immune reaction to bacteria is enhanced to improve the long term stability of the artificial tooth root.

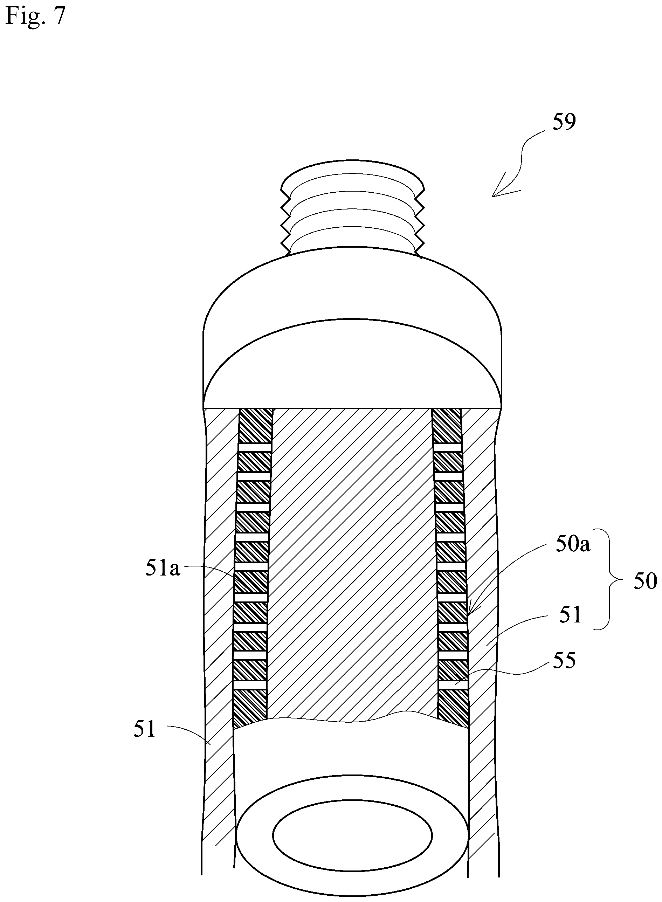

The artificial tooth root 59 shown in Figure 7 is the tooth root in which the supporting column is not provided inside of the substrate, and other configurations are substantially same with the artificial tooth root 50. Thereby, the strength of the artificial tooth root 59 is weakened compared with the artificial tooth root 50, but its mounting becomes easy.

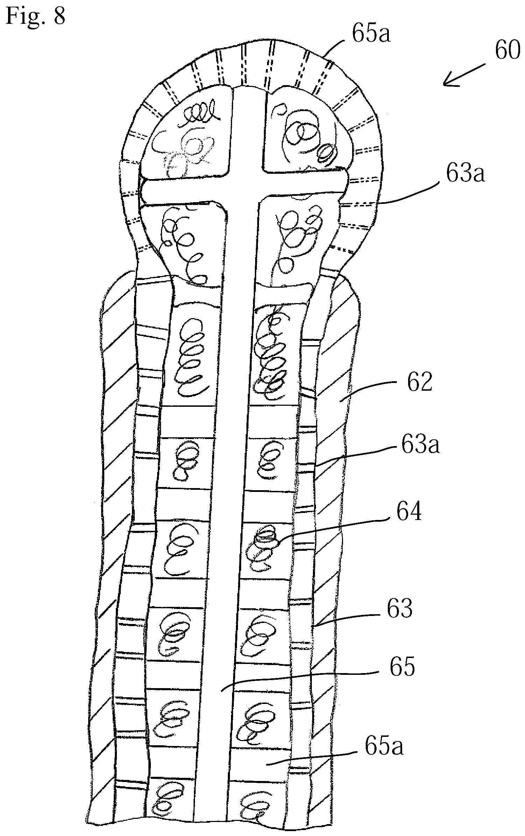

The scaffold material shown in Figure 8 is used for the artificial joint capable of inducing a biological hard tissue. This artificial joint 60 comprises a columnar substrate 61 and a cell induction layer 62 provided in the outer periphery of the substrate.

The substrate 61 comprises a substrate body 63 having a small hole 63a of the diameter 2 mm or less in its side, and the three dimensional porous structure 64 charged inside of the substrate body, and a supporting column 65 disposed along the inside of the substrate body 63. On the upper end of the supporting column 65, an artificial osteomere 65a is formed, and in the middle portion of the supporting column 65, a rib 65a protruding in the radial direction in the substrate is formed. And this artificial joint 65a may be provided with a small hole 63a as shown in the imaginary line. Further, the small holes 63a shown by this imaginary line, may be thinner micro holes.

The three dimensional porous structure 64 is substantially same with the three dimensional porous structure of Figure 6, and formed, for example, by charging the fiber of titanium metal inside of the substrate body 63.

As the material for the supporting column 65, titanium and titanium alloy having high strength and high biocompatibility are employed.

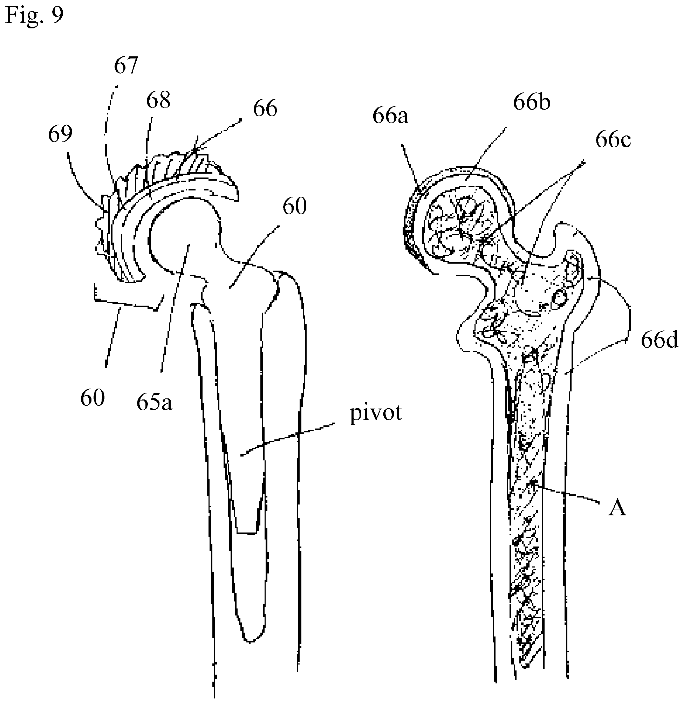

The artificial joint 60 thus configured is disposed as shown in Figure 9a. Here, Figure 9b is an outline drawing of a joint in a normal condition. The reference numeral 66b is a cartilage, the reference numeral 66c is a compact bone, the reference numeral 66d is a portion where the spongy bone and the bone marrow is mixed, and the reference numeral 66e presents the bone marrow. And the reference numeral 66 of Figure 9a is a tray of the artificial joint. This tray 66 comprises fan-shaped metal substrate 67, a synthetic resin layer 68 provided inside thereof, and a metal fiber layer 69 composed of titanium web provided outside of the metal substrate. As shown in Figure 9a, since the bone marrow A of a living body exists in the vicinity where the artificial joint 60 is disposed, the bone marrow A is induced from the lower end opening 61b to inside of the three dimensional structure 64 of the substrate. And then, the induced bone marrow A is sent to the entire area of the cell induction layer 62 through the small hole 63a of the substrate body. Thereby, the shortening of the treatment period of the artificial joint 60 can be attained. When the area of the bone regeneration is wide such as the artificial joint, it requires long time of treatment for the bone to infiltrate only from the outside. Therefore, the regeneration from inside is important, and the artificial joint of this invention 60 enables the regeneration process beginning with the inside. And, when it is integrated with the surrounding bones, since the blood vessels, nerves are induced and formed in the three dimensional structure, it is restored as a structure alike hitherto existing living body. Therefore, the bone induced in the regenerated cell induction layer 62 can receive the nutrition from the bone marrow and never thins down after a long period. Accordingly, the conventional problem of artificial joints, which is the loosening between the artificial joint and the bone, will not arises and it is not necessary to replace the artificial joint.

### Embodiments

### [Embodiment 1]

The artificial tooth root 40 shown in Figure 5 is fabricated. The female screw formed inside of the lead-in hole 44 is not formed. The substrate 41 is formed by molding and sintering titanium wires of the diameter 80 *µ* m to make the diameter of the flange portion 43 be 4 mm, the diameter of the lead-in hole be 2 mm, the length be 3 mm, and the average pore size be 80 *µ* m.

And, to form the cell induction layer 42, nonwoven fabrics formed by intertwining titanium metal wires of the diameter 80 *µ* m is wrapped so as to be the average pore size 200 *µ* m.

And then, the titanium rod is mounted to the lead-in hole 44 and they are jointed by vacuum sintering to fabricate the artificial tooth root. In the artificial tooth root 40 thus formed, the substrate 41 corresponds to the binding layer in the claim, and the cell induction layer 42 corresponds to the metal fiber layer.

### [Comparative example 1]

Metal wires of the diameter 80 *µ* m are entwined around a titanium rod and wrapped so as to be the average pore size 200 *µ* m. The artificial tooth root is fabricated by sintering this, which is made to be the comparative example 1.

### [Comparative example 2]

Metal wires of the diameter is 80 *µ* m are entwined around a titanium rod and wrapped so as to be the average pore size 80 *µ* m. The artificial tooth root is fabricated by sintering this, which is made to be the comparative example 1.

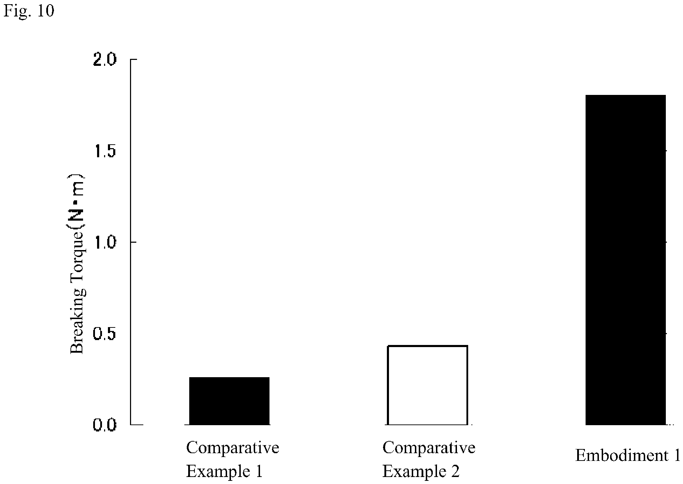

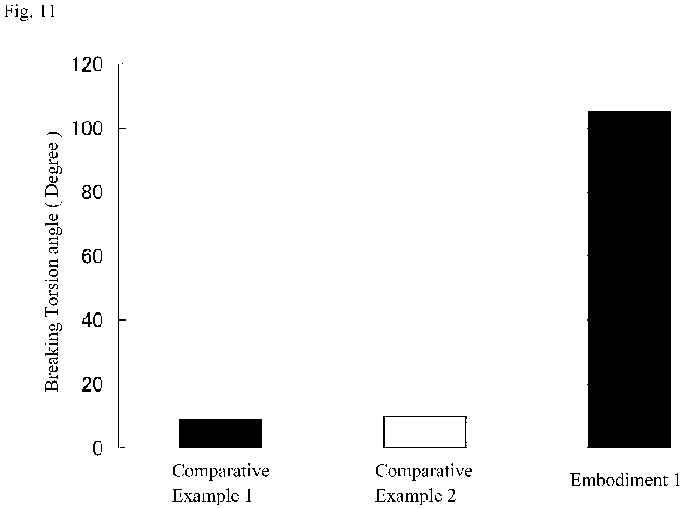

The torsion breaking test of the embodiment 1, the comparative example 1, and the comparative example 2 were performed. The results of this breaking torque and the breaking torsion angle are shown in Figure 10 and Figure 11. As found from these results, the embodiment 1 of the artificial tooth root of this invention did not break when four times of the breaking torque was applied. Further, in the embodiment 1, no breaking between the rod and the binding layer and the metal fiber layers was observed even against ten times of the torsion of the artificial tooth root of the comparative example 1, proving the high flexibility thereof.

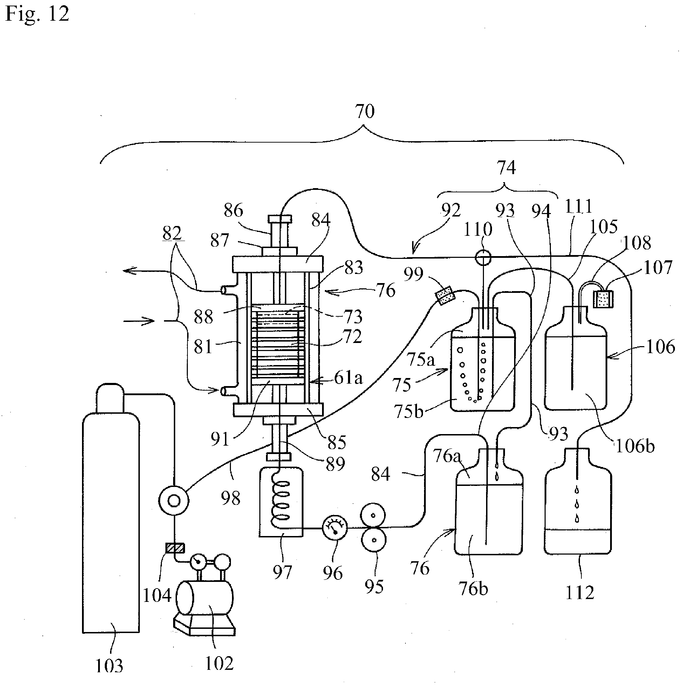

The producing apparatus 70 shown in Figure 12 is equipped with a column 71, a dish-like holder 72 accommodated inside of the column in a laminated state, a culture platform 73 accommodated in the holder, a closed conduit 74 for circulating the culture solution in the column 71. In the conduit 74, a mixing tank 75 for mixing and supplying air and flesh culture medium (culture solution), and a bubble removal tank 76 are interposed.

As the column 71, Falmacia chromatocolumn with a jacket etc. is used. The outer periphery of the column body 71a is surrounded by a jacket 81, and to the inlet provided under the portion of the jacket and the outlet provided on the upper portion, a conduit 82 to circulate water of the temperature, for example 37°C, is coupled. Thereby, the culture medium charged inside of the column 71 can be maintained at a temperature inside of a living body. The body 71a is equipped with a cylindrical peripheral wall 83, an upper cap 84 to close the upper end and a lower cap 85 to close the lower end, and whole of them is coupled in a liquid tight condition. The peripheral wall 83 is usually made of glass but it may be made of metal such as stainless etc. As the culture medium, any of culture medium used usually can be employed. For example a minimum required culture medium of Eagle and the culture medium having an improved composition can be also employed.

A pipe like outlet member 86 is passed through the upper cap 84 of the column 71, and the outlet member is fixed to the upper cap 84 by a circular plate-like fixing member 87. In the lower end of the outlet member 86, an upper holding member 88 to support the upper end of the laminated multistage holder 72 is fixed. On the other hand, in the lower end cap 85, a pipe-like inlet member 89 is passed through, and fixed to the lower cap 85 by the fixing member 90. In the upper end of the inlet member 89, a lower holding member 91 to support the lower end of the laminated holder 72 is fixed.

To the upper holding member 88, one end of a discharging tube 92 to draw off the used culture medium from the column 71 is coupled and goes out through the outlet member 86, and its other end opens at the upper cavity (vapor portion) 75a in the mixing tank 75. To the upper end of the outlet member 86, one end of the discharging tube 92 is coupled, and in the culture medium (liquid) 75b of the mixing tank 75, one end of a middle tube 93 is dipped, the other end of the middle tube opens at the upper cavity 76a of the bubble removal tank 76. In the culture medium 76b in the bubble removal tank 76, one end of a return tube 94 is dipped, and coupled to the lower end holding member 91 via a peristaltic pump (tubing pump) 95, a pressure gauge 96, and a preheating tank 97 through the inlet member 89 of the column 71.

The discharging tube 92, the middle tube 93, and the return tube 94 configures the above described conduit 74 to recycle the culture medium to the column 71, and the peristaltic pump 95 is to circulate the culture medium in the conduit 74. The peristaltic pump 95 serves as a sort of the heart in a living body. The preheating tank 97 is an apparatus to heat the culture medium being fed in the column 71 up to a temperature near that of a living body (for example 37°C).

In the culture medium 75b of the mixing tank 75, an end of a tube 98 for supplying mixing gas of air and carbon dioxide is dipped. The tube is coupled to a mixer 100 for mixing the air and the carbon dioxide via a filter 99. To the mixer 100, a compressor 102 and a gas container 103 for supplying carbon dioxide is coupled via a hose or a tube. The reference numeral 104 is a filter.

Further, in the upper cavity 75a of the mixing tank 75, the end of a tube 105 for supplying flesh culture medium opens, and the other end of the tube 105 is dipped in a culture medium 106b of the flesh culture medium tank 106 charged with a flesh culture medium 106b. The flesh culture medium tank 106 is kept airtight with a cap, and in the upper cavity 106a, a tube 108 opens to inlet ambient air via a filter 107.

Further, in this embodiment, a switching valve (cock) 110 is interposed in the midway of the discharging tube 92, and to the cock 110, a retrieving/monitoring tank 112 is coupled via a tube 111. The retrieving/monitoring tank 112 is the tank to make certain of producing the substance targeted by cells such as collagen after operating the production apparatus, and to retrieve the produced substance.

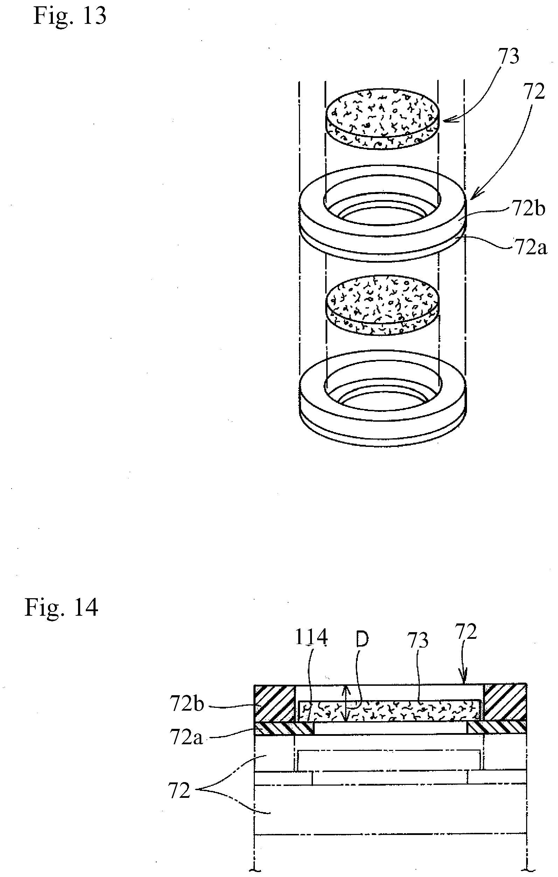

Figure 13 and Figure 14 are the magnified figure of the holder 72 accommodated in the column 71 of Figure 12 and the culture platform 73 accommodated therein. The holder 72 is a rough dish-like member presenting ring shape, and a step portion 114 to mount the culture platform 73 is provided therein. The depth D from the upper end of the holder 72 to the step portion 114 is deeper than the thickness of the culture platform 73. The culture platform 73 is a platform in which cells are seeded and cultured, and formed by a web composed by intertwining titanium or titanium alloy thin fibers into a mat-shape.

The thickness of the thin fibers composing the culture platform is preferable to be 200 *µ* m or less, particularly 100 *µ* m or less so as to construct a suitable space. Usually, that of 20 *µ* m or more, preferably 50 *µ* m or more is used. The length and the number of the thin fiber is not particularly limited. In addition, one culture platform 73 can be composed of one thin fiber. For intertwining the thin fiber, such a method etc. as to cross the thin fiber at right angles to form a metal mesh can be employed. And it can be composed also by making it be a nonwoven fabric or a woven sheet and punched out into a given shape. The thickness of the culture platform 73 is in the order of 1-2mm, and the diameter is different depending on the column (reference numeral 71 of Figure 3) to be used, and is usually in the order of 10-200mm. The weight of the culture platform is in the order of 1-100g. Other than titanium, for example, thin fibers of titanium alloy, stainless, gold, gold alloy etc can be used. In the thin fiber state or after the culture platform 73 is formed, the coating of calcium phosphate compound such as hydroxyapatite, hydroxyapatite containing apatite carbonate enhances the biocompatibility, which is preferable because cells or microorganisms are easy to adhere. Further, begin with collagen, the prior coating of cell adhesion substance such as fibronectin thereon to make, as it is called, "double coat " is extremely effective.

The holder 72 is formed using, for example, plastic such as polyacrylamid. When polyacrylamid is used, there is a merit of high workability. But other plastics such as polypropylene, polyethylene, and styrene can be used. In addition, it can be so composed that on a divider 72a composed of thin annular plate, a thick ring-like supporter 72b having the same outer diameter is superposed and adhered. When the culture platform 73 is let in and superposed on such holder 72, many culture platforms 73 can be laminated in a condition spaced mutually between the culture platforms 73. Therefore, when they are accommodated with this condition in the column 71 of Figure 12, the culture medium contacts each culture platform 73, enabling long term continuous culture of the cells cultured on the culture platform 73.

As the cells cultured by the culture platform 73, cells producing various cellular substances can be used as follows; the cells of anchorage dependence which can be cultured by making the culture platform 73 as an anchor, for example, cells producing collagen such as fibroblasts, osteoblasts; cells producing adrenocorticotrophin (hormone) such as vascular endothelial cells, adrenocortical cells; cells producing pancreatic cells such as insulin; bone marrow-derived hemocytes in the differentiation process of producing cytokine of blood system; cells producing valuable blood protein such as hepatocytes. However, the production apparatus of Figure 12 can be used not only to produce the cellular substances but also simply to culture/proliferate cells, in which case the retrieving/monitoring tank 52 is not necessary. In such a case that the production of cellular substances is not the object as the culture/proliferation of cells, it is not a producing apparatus but a simple cell culture apparatus. When collagen is produced, what containing vitamin C etc. is preferable as the culture medium. To avoid bovine spongeform encephalopathy (BSE), synthesized culture medium may be used.

Thus, on the culture platform 73 which has cultured cells (the first cell), different kind of the second cell may be seeded and cultured. Thereby, the culture platform itself which cultures cells of the two layers can be used as a three dimensional artificial model for medicine screening. Particularly, by seeding fibroblasts on the culture platform as the first cell, and by seeding and culturing epithelial cells as the second cell, a three dimensional skin model can be manufactured.

In this embodiment, the circular plate-like culture platform 73 is used, but when the three dimensional artificial model is manufactured, the shape is not particularly limited.

Then, the method using the production apparatus 70 of Figure 12 to produce collagen from collagen-productive cells such as fibroblasts osteoblasts etc. is described. Fibroblasts are produced as follows and used; for example, oral mucosa of a patient who has partial deficit of cartilage, periodontal tissue, nerve, muscle, and ligament is taken in the order of several mm, and fibroblasts contained therein are proliferated by a publicly known method. In addition, other than patients, fibroblasts separated from the umbilical cord of a specific neonate or subcutaneous tissue of a young adult can be separated and proliferated and used. The osteoblasts can be obtained from bone-marrow-derived mesenchymal stem cells.

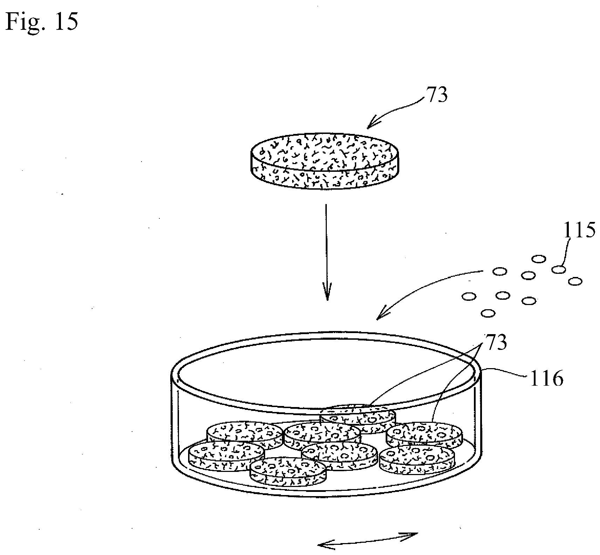

Then, as shown in Figure 15, a cell piece 115 of proliferated fibroblasts and osteoblasts is seeded in the culture platform 73 accommodated in a petri dish 116. The culture platform 73 in the petri dish 116 is dipped in a suitable culture medium. And then, the petri dish 116 is set in a rotation culture device to perform rotation culture which gives stimulus to cells by reciprocation and rotation. After making sure that the cell piece 115 has been cultured on the culture platform 73, as shown in Figure 13, the culture platform 73 is accommodated in the holder 72, and many holders 72 are let in the column 71 of Figure 12 in piles. The number of piled holders 72 is different depending on the quantity of the collagen to be produced, but it is in the order of 20-50 stages.

As described above, after many holders accommodating culture platforms 73 are set in the column 71 of Figure 12, the peristaltic pump 95 is operated to circulate the culture medium in the conduit 74, and the cells in the culture platform 73 is cultured. In the jacket of column 71, as described above, warm water of the temperature 37°C is circulated to make the environment inside of the column 71 be similar to that of a living body. To the mixing tank 75, air is supplied from the above compressor 102, and carbon dioxide is supplied from the gas container 103. The ratio of the both is, for example, carbon dioxide 95% (weight %), air 5% (weight %), and the amount of supply is in the order of 0.1-1 litters/minute. The mixed gas of carbon dioxide and air is mixed in the culture medium by bubbling from the end of the tube 98 for supply. This mixing tank 75 works as a sort of the lung of a living body.

From the flesh culture medium tank 106 to the mixing tank 75, flesh culture medium is supplied by the principle of siphon when the liquid level of the mixing tank 75 becomes low. But it can be forcedly supplied using a pump. The amount of the flesh circulating liquid supplied to the mixing tank 75 is in the order of 0.1-1 ml/minute. Since the supplied culture medium works so as to supply nutrition to the cells, it acts also as the digestive organ.

In the bubble removal tank 76, the culture medium sent from the mixing tank 75 is stored calmly, thereby, among the mixed gas of carbon dioxide and air mixed by bubbling in the culture mixing tank 75, what remaining as bubbles being not dissolved in the culture medium is made to be emerge into the upper cavity and removed.

By operating the producing apparatus 70 as above, the culture platform 73 accommodated in the column 71 secrets collagen. Among the collagen secreted, a part is secreted in the culture medium and others are deposited in the culture platform as insoluble collagen. In the culture medium, both of the soluble collagen and insoluble collagen are contained, those collagens are continuously retrieved into the retrieving/monitoring tank 112 by switching the cock 110 suitably. More specifically, from the retrieved culture medium in the retrieving/monitoring tank 112, collagen can be refined and separated by salting-out method, ion-exchange method etc.

On the other hand, the deposited insoluble collagen in the culture platform 73 can be retrieved almost completely as follows; the each culture platform removed from the column 71 is extracted using collagen-solubilization menstruum, for example, neutral buffered liquid containing 1 M salt , 0.5 M acetic acid, or 0.5 M acetic acid containing pepsin.

Using the producing apparatus 70 as described above, it is possible to produce collagen continuously by culturing fibroblasts obtained from patient's oral mucosa, and the both of soluble collagen and insoluble collagen can be efficiently retrieved. The retrieved collagen can be restored to the region needed for treatment of the patient such as bone, cartilage, periodontal tissue, nerve, muscle, and ligament. And, the collagen produced from tissues obtained from donors of the tissues other than patients can be used generally for the regeneration treatments of patients.

In this producing method described above, since the culture platforms 73 composed of intertwined three dimensional titanium thin fibers are made to produce while being superposed in several dozen piles and being attempted to separate respectively, the amount of production can be increased more than ten times compared with conventional flat plate methods. Thereby, from the cells in which collagen-producing gene is brought as a matter of course, without genetic manipulation, from normal human cells, a large amount of collagen can be produced to provide safe material for regeneration treatments.

In the previous embodiment, the case of producing collagen is described, the substantially the similar method can be used for producing cytokine such as bone morphogenetic protein, hormone such as insulin, micro activated substance such as interferon, and other recombinant protein valuable for medical treatments and industries. The case of using human cells is described above, but it is possible to produce collagen and other biological substances using cells of animals other than human. In this case, provided that safety is verified, it can be used for regeneration treatments for human and in the field of veterinary. Further, other than cells, in the culture of anchorage dependent microorganisms producing valuable biological substances and in the production of valuable substances by the micro organisms, the method and the apparatus of this invention can be employed obtaining similar effects.

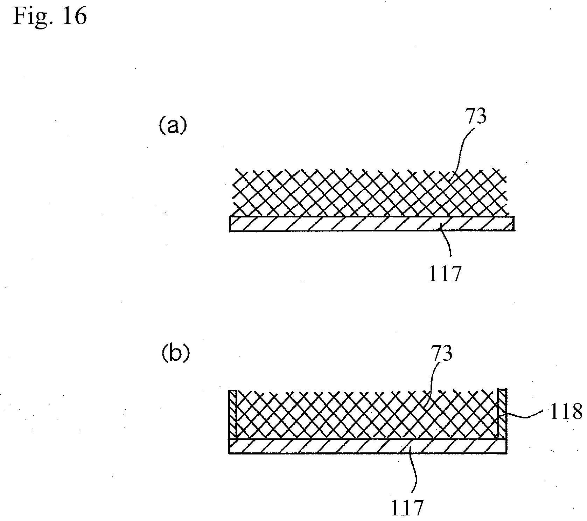

In the above embodiment, as the culture platform 73, the web composed by intertwining titanium or titanium alloy thin fibers so as to a form a mat-like shape is used as it is, but as shown in Figure 16a, it can be a complex layer by providing an impermeable layer 117 which has impermeability to culture solutions, cells, micro organisms or activated substances produced by these cells or microorganisms under the lower face of the culture platform 73. Such impermeable layer 117 can be formed of synthetic resin sheets, films, meal foils, and metal plates. Moreover, it can be provided separately to the culture platform 73, and in addition to this, it can be formed by providing a layer to yield, as it is called, plugging by charging powders, micro beads in the lower face of the culture platform 73.

As described above, when the impermeable layer 117 is provided in the lower face of the culture platform 73, cells and microorganisms become hard to fall down from the culture platform 73, thereby the usual shaking culture is not necessary and the culture in a culture dish is possible.

Further, as shown in Figure 16b, in addition to the bottom of the culture platform 73, the impermeable layer 118 may be also provided in the side of the culture platform 73. As this side impermeable layer 118, what is similar to the bottom impermeable layer described above can be used. When the impermeable layer 117, 118 are provided in the bottom and side, the activated substances secreted from the cells or microorganisms are not scattered about and easy to stay on the impermeable layer 117, thereby the cells or microorganisms are activated.

When the culture platform 73 is let in the petri dish 116 as shown in Figure 15, or let in the holder 72 as shown in Figure 13, 14, it is possible to let in the culture platform together with the impermeable layer 117, 118 as it is, thereby it brings about the effect that cells and microorganisms are hard to fall down. However, in the case that the impermeable layer 117, 118 are composed so as to be detachable to the culture platform 73, they can be made to be detachable in the specific condition when being let in the holder 72.

### [Embodiment 1]

A titanium web in which titanium metal wires of the diameter 50 *µ* m are intertwined so as to be the void ratio 87% corresponding to the culture platform used for the culture method of this invention was manufactured. This is made to be the embodiment 1.

### [Embodiment 2]

A culture platform in which the titanium web of the embodiment 1 is inserted to the bottomed cylindrical holder made of titanium is manufactured. This is made to be the embodiment 2.

### [Comparative Example 1]

A conventional publicly known plastic cell composed of plastic plane dish is made to be the comparative example 1.

Fibroblasts are seeded in the embodiment 1, 2, and the comparative example 1, and cultured two weeks while being dipped in the culture solution.

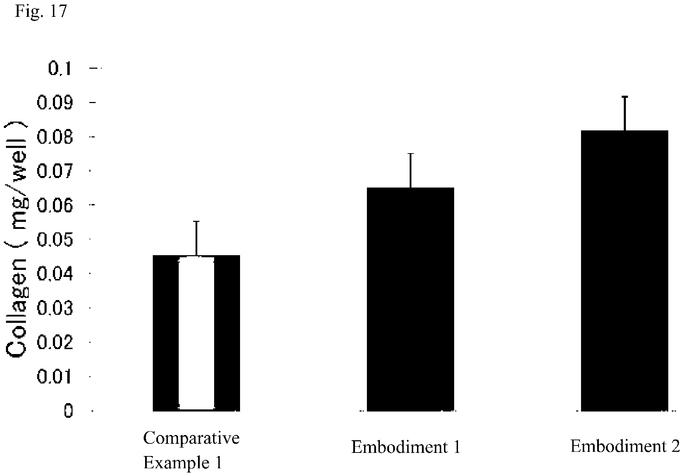

Figure 17 shows the amount of production produced by the fibroblasts at this time period. As found from the figure, when cultured using the culture platform used for this invention (Embodiment 1), the amount of production of collagen was elevated 1.4 times larger than that of the conventional plastic cell. Further, when cultured by the culture platform with the holder, the production of collagen was elevated to no less than 1.8 times.

## Claims

1. A scaffold material capable of inducing a biological hard tissue or soft tissue, comprising:
a metal substrate, and
a metal fiber layer composed of a metal wire prepared in an outer periphery of the metal substrate,
wherein the average pore size of the metal fiber layer is 100 to 400 *µ* m; and wherein the scaffold material further comprises:
a binding layer composed of a metal wire prepared in an inner periphery of the metal fiber layer,
wherein the average pore size of the binding layer is less than 100 *µ* m.

2. A scaffold material according to claim 1,
wherein an average pore size of the metal fiber layer is made to vary at a slant or in an incremental steps increasing from an innermost side to an outermost side.

3. A scaffold material according to claim 1,
wherein the metal fiber layer is formed by fixing a metal nonwoven fabric formed by intertwining a metal wire having a diameter of 5 to 400 *µ* m.

4. A scaffold material according to claim 2,
wherein the metal fiber layer is composed of the metal wire having a diameter of 5 to 400 *µ* m, and
wherein the average pore size of the metal fiber layer formed at the slant or in the incremental steps increasing from the innermost side to the outermost side is made according to a differences of a degree of the intertwining of the metal wires.

5. A scaffold material according to claim 2,
wherein the metal fiber layer is composed of the metal wire having a diameter of 5 to 400 *µ* m, and
wherein the average pore size of the metal fiber layer formed at the slant or in the incremental steps increasing from the innermost side to the outermost side is made according to a difference of the diameter of the metal wires.

6. A scaffold material according to claim 1,
wherein the binding layer is formed by intertwining a metal wire having a diameter of 5 to 400 *µ* m, and metal powders or metal particles are thickly implanted between the intertwined wires.

7. A scaffold material according to claim 6,
wherein a diameter of the metal powders or the metal particles are 100 *µ* m or less.

8. A scaffold material according to any of claims 1 to 7, further comprising;
a reinforcing layer composed of a metal wire having a diameter of 100 to 2000 *µ* m prepared in an outer periphery of the metal fiber layer,
wherein an average pore size of the reinforcing layer is 100 to 2000 *µ* m.

9. A scaffold material according to any of claim 1 to claim 8,
wherein the metal substrate is a rod in shape, and the rod comprises a bridge girder or a protrusion formed on a surface of the rod protruding outside against the radial direction of the rod.

10. A scaffold material according to any of claim 1 to claim 8,
wherein the metal substrate is a plate in shape and the plate comprises a protrusion protruding outward on a surface of the plate.

11. A scaffold material according to any of claim 1 to claim 10,
wherein the metal substrate and the metal fiber layer and/or the binding layer are fixed by sintering together.

12. A scaffold material according to claim 11,
wherein the sintering is performed at a temperature 0.3 to 0.9 times of a melting point (Tm·°C) of the metal substrate.

13. A scaffold material according to claim 1,
wherein the metal substrate and the metal fiber layer or the binding layer are bonded together with an adhesive material.

14. A scaffold material according to any of claim 1 to claim 13,
wherein the material of the metal substrate and the metal fiber layer and/or the binding layer is the one or two kinds of metal selected from a group composed of titanium, titanium alloy, gold, and gold alloy.

15. A scaffold material according to any of claim 1 to claim 14,
wherein the metal fiber layer and/or the binding layer is treated by apatite formation liquid and coated by hydroxyapatite containing apatite carbonate or other calcium phosphate compounds.

16. A scaffold material according to any of claim 1 to claim 15,
wherein the metal fiber layer and/or the binding layer is coated by one or two kinds or more of materials selected from groups composed of cytokine such as bone morphogenetic protein etc., hormone such as insulin etc., micro active substance such as interferon etc.

17. A biomedical implant material for implanting in a living body and integrally fixed to a peripheral surface of the living body, comprising:
a scaffold material capable of inducing a biological hard tissue or soft tissue according to any of claim 1 to claim 16.

18. A biomedical implant material according to claim 17,
used for a kind selected from groups composed of bone fixation fixtures such as artificial tooth roots, artificial joints, and bone plates, substitutional bones, artificial internal organs or their holding apparatuses, and skin terminals.

## Patentansprüche

1. Gerüstmaterial, das zum Induzieren von biologischem Hartgewebe oder Weichgewebe in der Lage ist, mit:
einem Metallsubstrat und
einer Metallfaserschicht, die sich aus einem Metalldraht zusammensetzt, der in einer äußeren Peripherie des Metallsubstrats vorbereitet ist,
wobei die mittlere Porengröße der Metallfaserschicht 100 bis 400 µm beträgt und wobei das Gerüstmaterial weiter aufweist:
eine Verbindungsschicht, die sich aus einem Metalldraht zusammensetzt, der in einer inneren Peripherie der Metallfaserschicht vorbereitet ist,
wobei die mittlere Porengröße der Verbindungsschicht weniger als 100 µm beträgt.

2. Gerüstmaterial nach Anspruch 1,
wobei eine mittlere Porengröße der Metallfaserschicht variabel gestaltet ist, um kontinuierlich oder in inkrementellen Schritten von einer innersten Seite zu einer äußersten Seite anzusteigen.

3. Gerüstmaterial nach Anspruch 1,
wobei die Metallfaserschicht gebildet ist, indem ein Metallfließstoff befestigt ist, der durch Verflechten eines Metalldrahts mit einem Durchmesser von 5 bis 400 µm gebildet ist.

4. Gerüstmaterial nach Anspruch 2,
wobei die Metallfaserschicht sich aus dem Metalldraht mit einem Durchmesser von 5 bis 400 µm zusammensetzt und
wobei die mittlere Porengröße der Metallfaserschicht, die kontinuierlich oder in inkrementellen Schritten von der innersten Seite zu der äußersten Seite ansteigend gestaltet ist, durch Unterschiede des Grades der Verflechtung der Metalldrähte erzeugt ist.

5. Gerüstmaterial nach Anspruch 2,
wobei sich die Metallfaserschicht aus Metalldraht mit einem Durchmesser von 5 bis 400 µm zusammensetzt und
wobei die mittlere Porengröße der Metallfaserschicht, die kontinuierlich oder in inkrementellen Schritten von der innersten Seite zu der äußerste Seite ansteigend gestaltet ist, durch Unterschiede des Durchmessers der Metalldrähte erzeugt ist.

6. Gerüstmaterial nach Anspruch 1,
wobei die Verbindungsschicht durch Verflechten eines Metalldrahts mit einem Durchmesser von 5 bis 400 µm hergestellt ist und wobei Metallpulver oder Metallteilchen dicht zwischen die verflochtenen Drähte eingebracht sind.

7. Gerüstmaterial nach Anspruch 6,
wobei der Durchmesser der Metallpulver oder der Metallteilchen 100 µm oder weniger beträgt.

8. Gerüstmaterial nach einem der Ansprüche 1 bis 7, weiterhin mit:
einer Verstärkungsschicht, die sich aus einem Metalldraht mit einem Durchmesser von 100 bis 2000 µm zusammensetzt, der in einer äußeren Peripherie der Metallfaserschicht vorbereitet ist,
wobei eine mittlere Porengröße der Verstärkungsschicht 100 bis 2000 µm beträgt.

9. Gerüstmaterial nach einem der Ansprüche 1 bis 8,
wobei das Metallsubstrat die Form eines Stabes hat und der Stab einen Brückenträger oder einen Vorsprung aufweist, der an einer Oberfläche des Stabes gebildet ist und nach außen gegen die radiale Richtung des Stabes vorsteht.

10. Gerüstmaterial nach einem von Anspruch 1 bis Anspruch 8,
wobei das Metallsubstrat die Form einer Platte hat und die Platte einen Vorsprung aufweist, der aus einer Oberfläche der Platte vorsteht.

11. Gerüstmaterial nach einem von Anspruch 1 bis Anspruch 10,
wobei das Metallsubstrat und die Metallfaserschicht und/oder die Verbindungsschicht verbunden sind, indem sie zusammengesintert sind.

12. Gerüstmaterial nach Anspruch 11,
wobei das Sintern bei einer Temperatur des 0,3 bis 0,9-fachen des Schmelzpunkts (Tₘ in °C) des Metallsubstrats durchgeführt ist.

13. Gerüstmaterial nach Anspruch 1,
wobei das Metallsubstrat und die Metallfaserschicht oder die Verbindungsschicht durch ein Haftmaterial miteinander verbunden sind.

14. Gerüstmaterial nach einem von Anspruch 1 bis Anspruch 13,
wobei das Material des Metallsubstrats und der Metallfaserschicht und/oder der Verbindungsschicht eine Art oder zwei Arten von Metallen ist, die aus der Gruppe bestehend aus Titan, Titanlegierung, Gold und Goldlegierung ausgewählt sind.

15. Gerüstmaterial nach einem von Anspruch 1 bis Anspruch 14,
wobei die Metallfaserschicht und/oder die Verbindungsschicht durch eine apatitbildende Flüssigkeit behandelt ist und durch ein Hydroxylapatit enthaltendes Karbonatapatit oder andere Kalziumphosphat-Verbindungen beschichtet ist.

16. Gerüstmaterial nach einem von Anspruch 1 bis Anspruch 15,
wobei die Metallfaserschicht und/oder die Verbindungsschicht durch eine Art oder zwei Arten oder mehr von Materialien beschichtet ist, die ausgewählt sind aus Gruppen bestehend aus einem Zytokin wie etwa knochenmorphogenetisches Protein etc., einem Hormon wie etwa Insulin etc., einer mikroaktiven Substanz wie etwa Interferon etc..

17. Biomedizinisches Implantatmaterial zum Implantieren in einen lebenden Körper und zum integralen Fixieren an einer äußeren Oberfläche des lebenden Körpers, mit:
einem Gerüstmaterial, das zum Induzieren von biologischem Hartgewebe oder Weichgewebe in der Lage ist, gemäß einem von Anspruch 1 bis Anspruch 16.

18. Biomedizinisches Implantatmaterial nach Anspruch 17,
das für eine Art verwendet wird, die aus den Gruppen ausgewählt ist bestehend aus Knochenfixierungs-Haltevorrichtungen wie etwa Kunstzahnwurzeln, künstliche Gelenke, und Knochenplatten, Ersatzknochen, künstlich innere Organe oder ihre Halteapparate und Hauttransplantate.

## Revendications

1. Matériau d'échafaudage capable d'induire un tissu dur ou un tissu mou biologique, comprenant :
un substrat métallique, et
une couche de fibres métalliques composée d'un fil métallique préparé dans une périphérie extérieure du substrat métallique,
dans lequel la taille de pore moyenne de la couche de fibres métalliques est de 100 à 400 µm ; et dans lequel le matériau d'échafaudage comprend en outre :
une couche de liaison composée d'un fil métallique préparé dans une périphérie intérieure du substrat métallique,
dans lequel la taille de pore moyenne de la couche de liaison est inférieure à 100 µm.

2. Matériau d'échafaudage selon la revendication 1,
dans lequel une taille de pore moyenne de la couche de fibres métalliques est amenée à varier en pente ou dans des pas incrémentaux augmentant d'un côté le plus à l'intérieur vers un côté le plus à l'extérieur.

3. Matériau d'échafaudage selon la revendication 1,
dans lequel la couche de fibres métalliques est formée en fixant un tissu non tissé métallique formé en entrelaçant un fil métallique ayant un diamètre de 5 à 400 µm

4. Matériau d'échafaudage selon la revendication 2,
dans lequel la couche de fibres métalliques est composée du fil métallique ayant un diamètre de 5 à 400 µm, et
dans lequel la taille de pore moyenne de la couche de fibres métalliques formée en pente ou dans les pas incrémentaux à partir du côté le plus à l'intérieur vers le côté le plus à l'extérieur est réalisée en fonction d'une différence d'un degré d'entrelacement des fils métalliques.

5. Matériau d'échafaudage selon la revendication 2,
dans lequel la couche de fibres métalliques est composée du fil métallique ayant un diamètre de 5 à 400 µm, et
dans lequel la taille de pore moyenne de la couche de fibres métalliques formée en pente ou dans les pas incrémentaux augmentant à partir du côté le plus à l'intérieur vers le côté le plus à l'extérieur est réalisée en fonction d'une différence du diamètre des fils métalliques.

6. Matériau d'échafaudage selon la revendication 1,
dans lequel la couche de liaison est formée en entrelaçant un fil métallique ayant un diamètre de 5 à 400 µm, et des poudres métalliques ou des particules métalliques sont implantées de manière épaisse entre les fils entrelacés.

7. Matériau d'échafaudage selon la revendication 6,
dans lequel un diamètre des poudres métalliques ou des particules métalliques est de 100 µm ou moins.

8. Matériau d'échafaudage selon l'une quelconque des revendications 1 à 7, comprenant en outre :
une couche de renfort composée d'un fil métallique ayant un diamètre de 100 à 2000 µm préparé dans une périphérie extérieure de la couche de fibres métalliques,
dans lequel une taille de pore moyenne de la couche de renfort est de 100 à 2000 µm.

9. Matériau d'échafaudage selon l'une quelconque des revendications 1 à 8,
dans lequel le substrat métallique est en forme de tige, et la tige comprend une poutre principale ou une protubérance formée sur une surface de la tige dépassant à l'extérieur contre la direction radiale de la tige.

10. Matériau d'échafaudage selon l'une quelconque des revendications 1 à 8,
dans lequel le substrat métallique est en forme de plaque et la plaque comprend une protubérance dépassant vers l'extérieur sur une surface de plaque.

11. Matériau d'échafaudage selon l'une quelconque des revendications 1 à 10,
dans lequel le substrat métallique et la couche de fibres métalliques et/ou la couche de liaison sont fixés ensemble par frittage.

12. Matériau d'échafaudage selon la revendication 11,
dans lequel le frittage est réalisé à une température de 0,3 à 0,9 fois un point de fusion (Tm·°C) du substrat métallique.

13. Matériau d'échafaudage selon la revendication 1,
dans lequel le substrat métallique et la couche de fibres métalliques ou la couche de liaison sont liés ensemble avec un matériau adhésif.

14. Matériau d'échafaudage selon l'une quelconque des revendications 1 à 13,
dans lequel le matériau du substrat métallique et de la couche de fibres métalliques et/ou de la couche de liaison est un ou deux types de métaux sélectionnés à partir d'un groupe composé du titane, d'un alliage de titane, de l'or, et d'un alliage d'or.

15. Matériau d'échafaudage selon l'une quelconque des revendications 1 à 14,
dans lequel la couche de fibres métalliques et/ou la couche de liaison est traitée par un liquide de formation apatite et revêtue par de l'hydroxyapatite contenant du carbonate d'apatite ou d'autres composés de phosphate de calcium.

16. Matériau d'échafaudage selon l'une quelconque des revendications 1 à 15,
dans lequel la couche de fibres métalliques et/ou la couche de liaison est revêtue par un ou deux types ou plus de matériaux sélectionnés à partir de groupes composés d'une cytokine telle qu'une protéine morphogénétique osseuse etc., d'une hormone telle que l'insuline etc., d'une substance micro-active telle que l'interféron.

17. Matériau d'implant biomédical destiné à une implantation dans un corps vivant et fixé intégralement sur une surface périphérique du corps vivant, comprenant :
un matériau d'échafaudage capable d'induire un tissu dur ou un tissu mou biologique selon l'une quelconque des revendications 1 à 16.

18. Matériau d'implant biomédical selon la revendication 17,
utilisé pour un type sélectionné à partir de groupes composés de dispositifs de fixation d'os tels que des racines de dents artificielles, des articulations artificielles, et des plaques osseuses, des os de substitution, des organes internes artificiels ou leurs dispositifs de support, et des bornes pour la peau.
